# EUROPEAN PATENT SPECIFICATION

(11) **EP 4 057 981 B1**
(45) Date of publication and mention of the grant of the patent: **02.07.2025**
(21) Application number: 20803611.1
(22) Date of filing: 13.11.2020
(51) Int. Cl.: A61J 1/20, A61M 39/10, A61M 39/22, A61M 39/00

(54) **ADAPTER FOR A DRUG MIXING AND INJECTION SYSTEM**
ADAPTER FÜR ARZNEIMITTELMISCH- UND -INJEKTIONSSYSTEM
ADAPTATEUR POUR SYSTÈME D'INJECTION ET DE MÉLANGE DE MÉDICAMENTS

(30) Priority: 13.11.2019 EP 19208867
(43) Date of publication of application: 21.09.2022
(73) Proprietor: Activoris Medizintechnik GmbH, 35285 Gemünden (Wohra) (DE)
(72) Inventor: FISCHER, Axel, Hans-Otto, 34630 Gilserberg (DE); NALIK, Olaf, 34508 Willingen (Upland) (DE); PREYSING, Regina, 34537 Bad Wildungen (DE); WEIDLER, Marion, 35321 Laubach-Lauter (DE)
(74) Representative: Synergy IP Group AG
(86) International application number: PCT/EP2020/082076
(87) International publication number: WO 2021/094548

(56) References cited:
- EP-B1- 2 337 543
- WO-A1-2013/128444
- WO-A1-2018/232171
- US-A1- 2010 305 507
- US-A1- 2013 046 270

## Description

The invention relates to a drug mixing and injection system in general and, in particular, to an adapter for said system facilitating the preparation of drug formulations for administration to patients.

### BACKGROUND OF THE INVENTION

Drugs intended for administration to patients are often provided either as dry powders (e.g. lyophilisates) or in liquid form. These drugs in powdered or liquid form typically require reconstitution with a pharmaceutically acceptable liquid, or medium (for instance, a solvent such as water for injection purposes), prior to administration, the pharmaceutically acceptable liquid being provided in a separate vessel than the drug. Reconstitution of the drug for adminstration to the patient should be performed with as little loss as possible and by transferring the pharmaceutically acceptable liquid under sterile conditions to the drug and to mix the drug with the pharmaceutically acceptable liquid.

Most systems from prior art require a multitude of handling steps in order to prepare the system for mixing and injection prior to use, such as breaking an ampoule containing the injectable medium, aspiration of the injectable medium into a syringe, transfer of the aspired medium to a drug vial and often several further steps to mix the drug with the injectable medium and in most cases dissolve it therein (e.g. create physical agitation by either shaking the vial and/or by transferring the drug-medium blend back and forth between the vial and the syringe). If the resulting drug formulation is intended for injection into a patient (rather than e.g. some test device), it is then often required to change the needle attached to the syringe prior to injection, since the needle diameters used for convenient handling and mixing are often larger than those allowing for easy and painfree injection. In most of these prior art systems, there is no direct connection between the drug contanining vial and the syringe, and thus any transfer of either the injectable liquid and/or the drug formulation resulting from mixing the drug with said injectable liquid involves air contact and in consequence contact with air-borne microbes. Since many users, or patients, are hesitant to open glass ampoules and preparing injectable drug formulations by themselves, all the above steps are often carried out by medically trained persons. In summary, the prior art devices, and in particular the multiple steps required with them, are not only susceptible to contamination but also not very user-friendly.

Recent approaches comprise systems for drug preparation and delivery that connect the vial containing the injectable medium with the drug vial and, optionally, with a delivery means, such as syringes. Such a system is disclosed in US20130046270A1 that describes a transfer device that comprises a least two containers, one containing an injectable medium and the second containing a drug, and a horizontally oriented delivery means with a plunger. Fluid connection between the two containers and the delivery means can be enabled by a slidable lock mechanism. However, as the transfer device of US20130046270A1 uses vacuum preconditioned in the drug container to navigate, or draw, the injectable medium from the first container to the drug container, the device has no system for repeating transfer steps of the injectable medium to the drug container and back that are necessary for drugs with low solubility. Therefore, it does not seem to be suitable for drugs with low solubility or for any other applications were repeated mixig steps are needed. For drugs with low solubility a monitoring of the degree of the solving state as well as repetitive flushing of the container with the drug are needed.

WO2013128444A1 discloses a two way fluid control device for use with a liquid vial, a drug vial under negative pressure and a needless syringe for preaparation of a liquid drug in the drug vial for inline aspiration to the needless syringe for administration purposes.

Both systems, the adapter of WO2013128444A1 and the transfer device of US20130046270A1, involve at least two vials, one for the provision of an injectable medium and a second for the provision of a medicament, usually in powdered form. In addition, there is a delivery device involved, in most cases a syringe. Thus, use of these systems includes a relatively high consumption of material, e.g. in form of vials.

Furthermore, the use of systems like the adapter of WO2013128444A1 and the transfer device of US20130046270A1 still requires multiple steps prior to administration to a patient that include the need for disconnecting the delivery device from the transfer device. This step is a potential source of incursion for contaminations as the syringe is exposed to ambient air.

It is thus an object of the present invention to provide devices that avoid these drawbacks of the prior art; for instance, devices that are easy-to-use (e.g. single handedly), less susceptible to tampering or even tamper-proof, which have a low material consumption and which reduce the contamination risk during aseptic preparation of a drug for administration to a minimum.

### SUMMARY OF THE INVENTION

The present invention provides a drug mixing and injection system, in particular an adapter for said system enabling the preparation of drug formulations.

In a first aspect, the invention relates to an adapter for a drug mixing and injection system comprising a first subunit (110) adapted to hold a vial (113) and a reservoir (115) with injectable liqid, a second subunit (120) comprising a valve (121) and an outlet port (122), an internal adapter channel (131), a first cannula (134) between the vial (113) and the internal adapter channel (131), wherein the adapter (100) has a resting position and an activated position.

In a second aspect, the invention relates to a drug mixing and injection system (200) comprising the adapter of the first aspect of the invention, a vial and a reservoir with injectable liquid.

In a third aspect, the invention relates to a method of preparing an injectable drug formulation by use of the drug mixing and injection system (200) of the above mentioned aspect of the invention comprising activation of the system and transferring an injectable liquid from the reservoir to the vial, mixing the drug and transfer the resulting injectable drug formulation back to the reservoir, bringing the valve to a second position and transferring the injectable drug formulation to the outlet port.

Further objects, aspects, useful embodiments, applications, beneficial effects and advantages of the invention will become apparent on the basis of the description of the invention, the examples, figures and claims below.

### DESCRIPTION OF THE FIGURES

### List of reference signs:

| | |
|---|---|
| Adapter (100) | Drug mixing and injection system (200) |
| First subunit (110) | Second subunit (120) |
| First opening (111) | Second opening (112) |
| Bottom wall of the first opening (111a) | Sidewall surrounding the first opening (111b) |
| Valve (121) | Valve knob (121a) |
| Vial (113) | Pierceable lid (114) |
| Vial locking means (116) | Vial locking hook (116a) |
| Outlet port (122) | Dedicated site for resting a finger (123) |
| Reservoir (115) | Housing of the reservoir (117) |
| Piston of the reservoir (118) | Internal adapter channel (131) |
| Parts of the internal adapter channel (131a, 131b, 131c) | Internal valve channels (131d) |
| Guiding means (132) | Subunit locking means (133) |
| Subunit retaining hooks (133a) | Subunit retaining grooves (133b) |
| First cannula (134) | Second cannula (135) |
| Connector in the second opening (136) | Bottom wall of the second opening (112a) |
| Vial bottom (113a) | Human hand (300) |

The following examples and figures serve to illustrate the invention in exemplary embodiments; however, they should not be understood as restricting the scope of the invention. Any reference signs used in the claims or throughout the description should not be construed as a limitation to the embodiments represented in any of the figures.

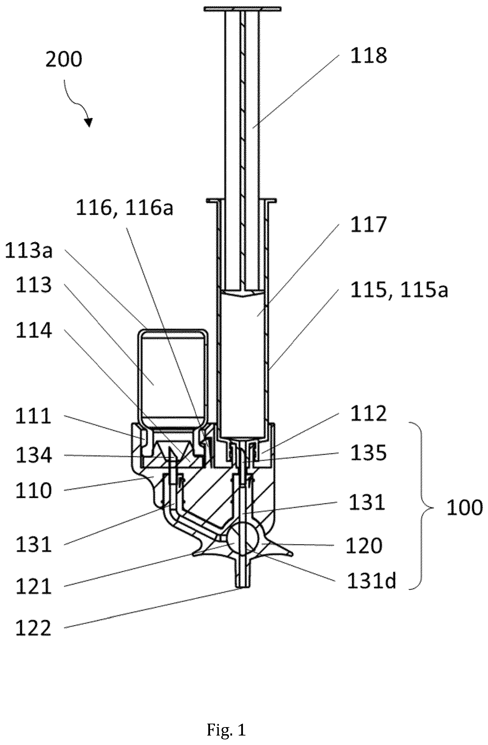

Figure 1 depicts an exemplary embodiment of the drug mixing and injection system (200) according to second aspect of the invention in a cross-sectional front view in the activated position of the adapter (100). The system is shown in the normal operational, upright position and comprises besides the adapter (100), the vial (113) connected to the first opening (111) of the first subunit (110), the reservoir (115) in form of a syringe (115a) connected to the second opening (112) of the first subunit (110) and the valve (121) as part of the first subunit (120).

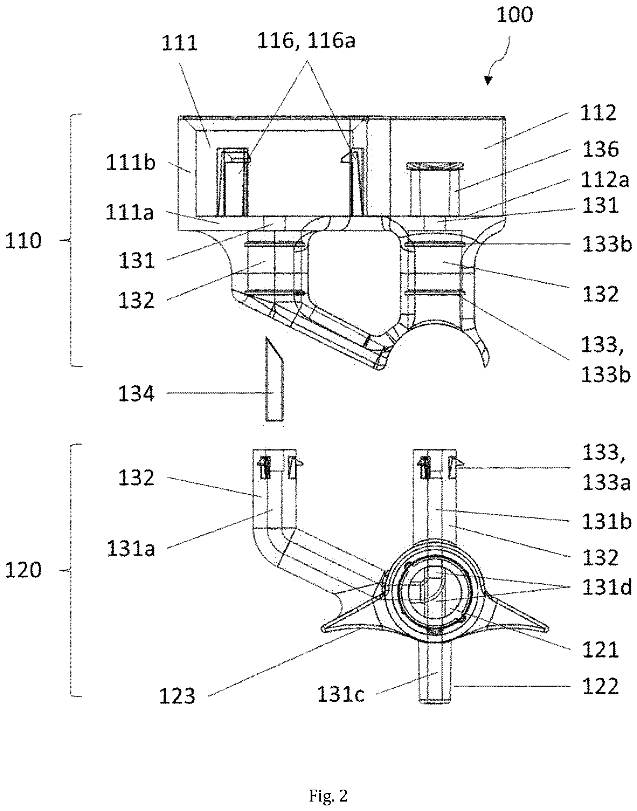

Figure 2 shows an exploded view of an exemplary embodiment of the adapter (100) for the drug mixing and injection system (200) with the first subunit (110) and the second subunit (120) and further components described in detail below. The adapter (100) of figure 2 is able to unreleasably connect the first and the second subunits (110, 120) in the resting as well as in the activated position due to subunit locking means (133) in form of subunit retaining hooks (133a) and corresponding subunit retaining grooves (133b).

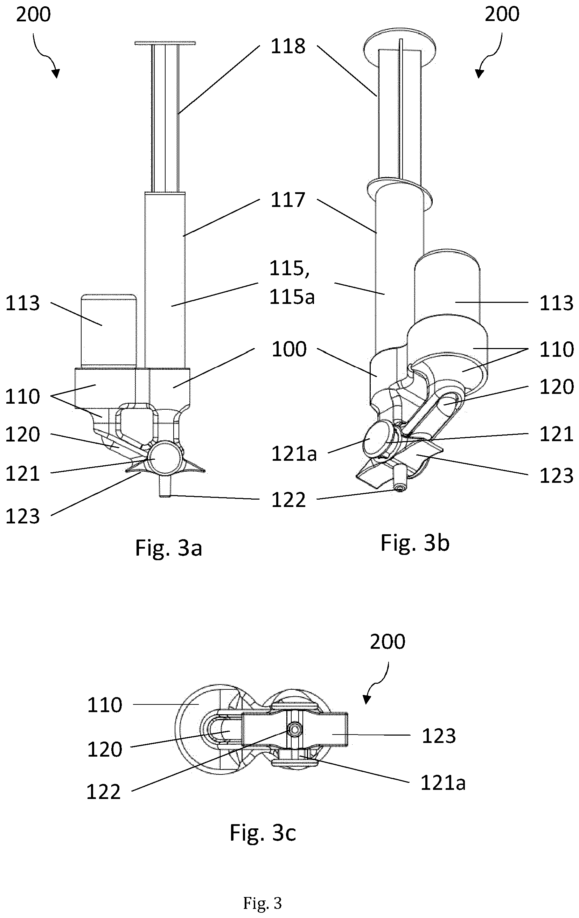

Figure 3 shows an exemplary embodiment of the drug mixing and injection system (200) from different perspectives, namely from the front (figure 3a), tilted from below (figure 3b) and bottom view (figure 3c).

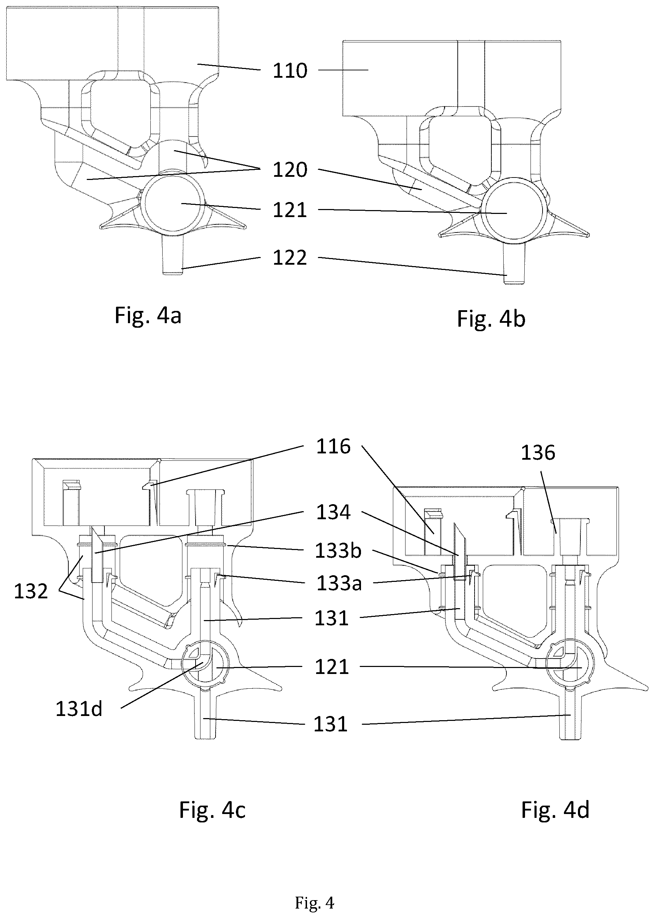

Figure 4 depicts an exemplary embodiment of the adapter (100) in the resting and the activated position. Figure 4a (plan view) and 4c (sectional view) show the adapter (100) in the resting position. Figure 4b (plan view) and 4d (sectional view) show the adapter (100) in activated position.

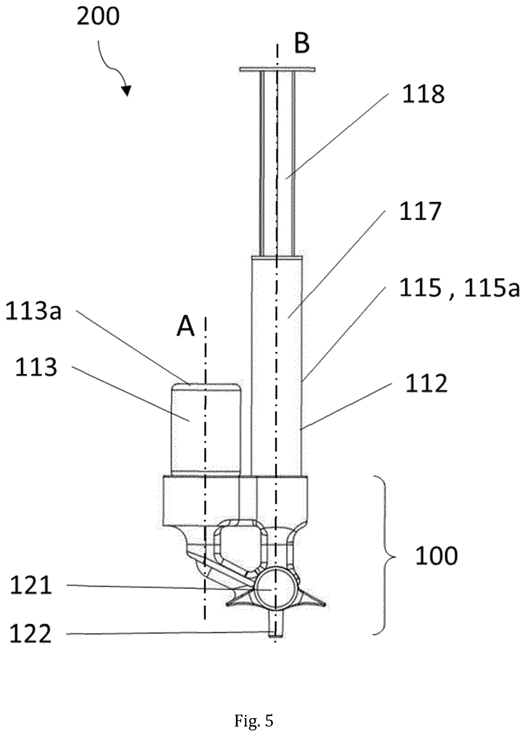

Figure 5 depicts an exemplary embodiment of the drug mixing and injection system (200) with the first and the second longitudinal central axes of the vial and the reservoir (A and B, respectively) positioned at an angle of ca. 0°, or in other words, being parallel to eachother.

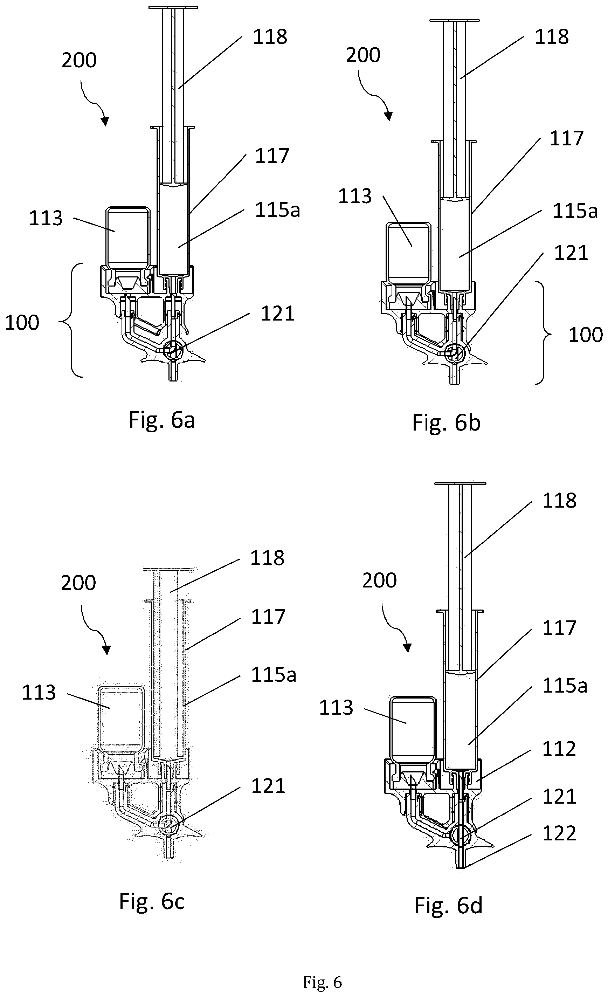

Figure 6 shows an exemplary embodiment of the drug mixing and injection system (200) during the different steps of the method of preparing an injectable drug formulation. Figure 6a: adapater (100) in resting position; Figure 6b: adapater (100) in activated position; Figure 6c: adapater (100) in activated position with the injectable liquid transfered to the vial; Figure 6d: adapater (100) in activated position with the resulting mixture transferred to the reservoir and thus ready to use.

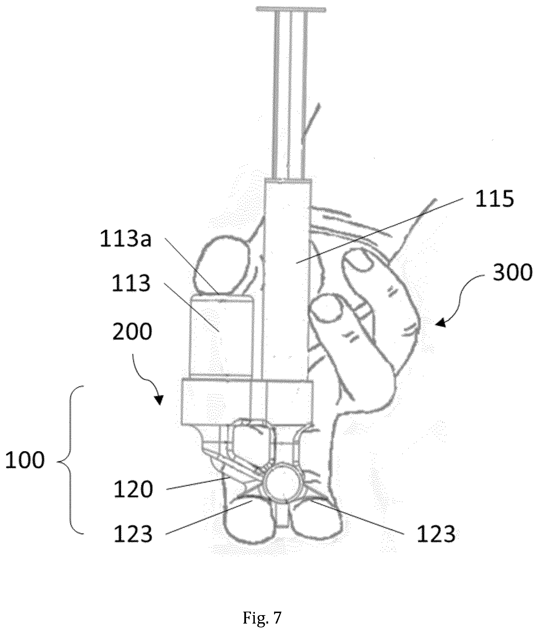

Figure 7 shows an exemplary single-handed use of the adapter (100) and the drug mixing and injection system (200) during the activation step. In the embodiment of figure 7, when activating the drug mixing and injection system (200) such that the adapter (100) is transferred from the resting position into the activated position, two fingers of the human hand (300) are positioned on the two dedicated sites for resting a finger (123), and the thumb is positioned on the vial bottom (113a).

### DEFINITIONS

The following expressions as used herein should normally be interpreted as outlined in this section, unless the description provides a different meaning in a specific context.

The terms "a", "an" or "the" do not exclude a plurality; i.e. these singular forms should be understood such as to include plural referents unless the context clearly indicates or requires otherwise. In other words, all references to singular characteristics or limitations of the present disclosure shall include the corresponding plural characteristic or limitation, and vice versa, unless explicitly specified otherwise or clearly implied to the contrary by the context in which the reference is made. The terms "a", "an" or "the" thus have the same meaning as "at least one" or as "one or more" unless defined otherwise.

The expressions "one embodiment", "an embodiment", "a specific embodiment" and the like mean that a particular feature, property or characteristic, or a particular group, or combination, of features, properties or characteristics, as referred to in combination with the respective expression, is present in at least one of the embodiments of the invention. The occurrence of these expressions in various places throughout this description do not necessarily refer to the same embodiment. Moreover, the particular features, properties or characteristics may be combined in any suitable manner in one or more embodiments.

Terms such as "about", "approximately", "ca.", "essentially", or "substantially" are meant to compensate for the variability allowed for in the technical field concerned and inherent in the respective products (e.g. in the pharmaceutical industry and in pharmaceutical products), such as differences in content due to manufacturing variation and/or timeinduced product degradation. The terms in connection with an attribute or value include the exact attribute or the precise value, as well as any attribute or value typically considered to fall within a normal range or variability accepted in the technical field concerned.

The term "comprise" is to be construed in an open and inclusive sense, as "including, but not limited to". The expressions "substantially consisting of" or "essentially consisting of" mean that no further components are added other than those listed.

The term "unreleasable" is understood to mean that the respectively connected components of the adapter or the drug mixing and injection unit are not releasable, or separable, from one another without requiring brute force and/or without breaking the adapter or the drug mixing and injection system.

The term "injectable liquid" is understood as any fluid that is able to flow and that is suitable for parenteral administration to a patient or animal. In one embodiment of the invention, the injectable liquid is a pharmaceutically acceptable liquid, like for example a salt solution or pure water. More specifically, the pharmaceutically acceptable liquid is water for injection (aqua ad injectabilia). In one embodiment of the invention, the injectable liquid can comprise further ingredients.

The terms "drug", "active agent", "therapeutic agent", "active pharmaceutical ingredient" (API), "active principle", or "bioactive agent" are used synonymously and refer to a compound or combination of compounds which are pharmaceutically active against an undesired condition.

The term "pharmaceutically acceptable" means that a material is useful in preparing a pharmaceutical composition that is generally safe, non-toxic and exhibits neither biologically nor otherwise undesirable properties which would prevent, or exclude, it from pharmaceutical use in humans.

Terms designating a position, orientation or direction, such as left, right, front, rear, back, top, bottom, up, down and the like, should be understood with reference to the orientation of the disclosed devices or their components under normal operational conditions, and typically from the perspective of the user.

A single unit may fulfill the functions of several features recited in the claims.

### DETAILED DESCRIPTION OF THE INVENTION

In one aspect, the invention provides an adapter (100) for a drug mixing and injection system comprising
a first subunit (110) comprising
   a first opening (111) adapted to hold a vial (113), said vial (113) comprising a pierceable lid (114), and exhibiting a first longitudinal central axis (A), and
   a second opening (112) adapted to hold a reservoir (115) comprising an injectable liquid, and exhibiting a second longitudinal central axis (B),
   wherein the first and the second openings (111, 112) are arranged to hold the vial (113) and the reservoir (115) in a position with the first and the second longitudinal central axes (A, B) positioned at an angle below 75°;
a second subunit (120) comprising a valve (121) and an outlet port (122);
   at least one internal adapter channel (131) enabling fluid connection between the first opening (111), the second opening (112), and the outlet port (122);
   wherein the valve (121) is adapted to switch at least between
   a first position enabling a first fluid flow between the first opening (111) and the second opening (112), and blocking the fluid connection to the outlet port (122), and
   a second position enabling a second fluid flow between the second opening (112) and the outlet port (122), and blocking the fluid connection to the first opening (111);
a first cannula (134) positioned between the valve (121) and the first opening (111), said first cannula (134) being adapted to pierce the lid (114) of the vial (113) and to enable fluid connection between the vial (113) and the at least one internal adapter channel (131), and
wherein the first and the second subunits (110, 120) are engageable with each other, in such a way that the adapter (100) has
a resting position in which the first and the second subunits (110, 120) are releasably or unreleasably connected and the first cannula (134) does not pierce through the lid (114) of the vial (113); and
an activated position in which the first and the second subunits (110, 120) are shifted towards each other and are in a position in which the first cannula (134) pierces through the lid (114) of the vial (113), thereby enabling fluid connection between the vial (113) and the at least one internal adapter channel (131).

According to the above aspect of the invention, the adapter (100) is a device that connects a vial (113) comprising a drug that needs to be mixed or reconstituted before administration to a patient with a reservoir (115) comprising an injectable liquid in that the drug is dissolved or with that the drug is mixed with. On the other hand, the adapter also represents an attachement to an injection means with that the reconstituted drug formulation is administered to a patient.

The adapter comprises two subunits. The first subunit (110) is the upper of the two subunits when the adapter (100) is held in the normal operational, upright position with the outlet port (122) showing downwards, as depicted in figure 1 which shows an example of the drug mixing and injection system (200) in the upright position. The first subunit (110) comprises a first opening (111), said first opening (111) being adapted to receive and hold a vial (113) which comprises a pierceable lid (114) and exhibits a first longitudinal central axis (A) as exemplarily depicted in figure 5. The first opening (111) comprises a bottom wall (111a) and a sidewall (111b) surrounding the first opening (111) that can receive the vial (113). The bottom wall (111a) has an opening, optionally in the middle of the bottom wall (111a), that is in fluid connection to the internal adapter channel (131). In one embodiment of the invention, the cross-section of the sidewall (111b) surrounding the first opening (111) is annular and the vial (113) has a corresponding round cross-section, at least at the vial opening.

In one embodiment of the invention, the first opening (111) is equipped with a vial locking means (116) for unreleasably connecting the vial (113) to the first opening (111). This is of particular advantage when drugs shall be mixed and administered that require a dosage accuracy like a specific dosage amount or underlye prescription restrictions. In this embodiment, the vial (113) cannot be removed and thus the drug is not available for tampering. The vial locking means (116) is any suitable means, like e.g. hooks, rings, protrusions or notches, that can interact with corresponding structures of the vial (113). In one of the preferred embodiments, the vial locking means (116) is adapted to unreleasably connect the first opening (111) with the vial (113) with a click mechanism, a pawl mechanism, or a snap-fit mechanism. In a preferred embodiment, the vial locking means (116) is provided in the form of at least one vial locking hook (116a), more preferably the vial locking means (116) is provided in the form of 2 - 6 vial locking hooks (116a), as exemplarily shown in figure 2. This is of advantage in cases where the vial (113) has a crimped neck that is narrower than the finishing part at the vial opening that has a collar-like rim which gives hold to the vial locking hook (116a) as depicted in figure 1. In these cases, the vial locking means (116) can catch or fasten behind the collar-like rim and provide an unreleasable connection with the vial (113).

In another embodiment of the invention, the first opening (111) and/or the vial locking means (116) is adapted to specifically connect to vials having a specific shape complementary to the first opening (111) and/or the vial locking means (116); for instance, the vial (113) and the vial locking means (116) may form a keylock with one of them providing the male part of the keylock and the respective other providing the female part of the keylock. In specific embodiments, these specific shapes do not have the usual round form but may be oval, angular, like e.g. triangular, or other forms. Alternatively, the neck of vial (113) can have a specific form, e.g. the can be crimped to a certain pattern, while the vial locking means (116) has the matching pattern. This is even more advantageous when mixing and administering drug subject to restrictions as it reduces the risk of confusion.

The vial (113) can be provided in any suitable form or shape known to the skilled person to be suitable for housing a drug; for example, in the form of a flask, a bottle, a tube, or a cylinder. According to the invention, the vial (113) comprises a pierceable lid (114), as exemplarily shown in figure 1. The pierceable lid (114) can be provided in any form or shape known to the skilled person to be suitable for closing, preferably aseptically closing, the vial (113) leaktight during storage and/or shipment and to be suitable to be perforated by a sharp and solid means, like e.g. a cannula or a needle. Typically, the pierceable lid (114) is provided in a round and planar form, i.e. the form of a disc. Preferably, the form of the pierceable lid (114) is such that there is a thicker part surrounding a thinner part in the middle of the pierceable lid (114), the thinner part adapted so as to be more readably pierceable. In one embodiment of the invention, the pierceable lid (114) comprises two separate parts, optionally made of different materials, one providing the thicker part and another providing the thinner part.

According to the invention, the vial (113) has a vial bottom (113a), a sidewall, and a vial opening covered and closed by the pierceable lid (114) opposite to the vial bottom (113a). According to the invention, the vial (113) also exhibits a first longitudinal central axis (A). The first longitudinal central axis (A) reaches from the vial bottom (113a) to the opening of the vial (113). In most cases, when the vial (113) has a cylindric shape as exemplarily depited in figure 7, the first longitudinal central axis (A) can be described as being parallel to the sidewall of the vial (113). The vial bottom (113a) may also be the position where a finger or a thumb of a human hand (300) can be positioned, or rested, when the adapter (100) or the drug mixing and injection system (200) is used single-handedly (see figure 7). The vial (113) is usually made of glass or plastic. In one embodiment of the invention, the vial (113) is a standard crimp neck vial, such as those used in many autosampling devices and/or for lyophilisation purposes.

The pierceable lid (114) can be made of any material known to the skilled person to be suitable for closing, preferably aseptically closing, the vial (113) leaktight during storage and shipment and to be suitable to be perforated by a sharp and solid means like e.g. a cannula or a needle. In a preferred embodiment, the pierceable lid (114) is made of plastic or rubber, more preferably of thermoplastic.

In one embodiment of the invention, the vial (113) is held under vacuum or under reduced pressure; e.g. a reduced pressure is below atmospheric pressure. This is of advantage as no or only little pressure compensation is necessary when transferring the injectable liquid from the reservoir (115) into the vial (113); in other words, the injectable liquid can be transferred from the reservoir (115) into the vial (113) with no or only little counterpressure.

The first subunit (110) further comprises a second opening (112) adapted to receive and hold a reservoir (115) comprising an injectable liquid, and exhibiting a second longitudinal central axis (B), as exemplarily depicted in figure 5. The second opening (112) comprises a bottom wall and a sidewall surrounding the second opening that can receive the reservoir (115). The bottom wall has an opening, preferably in the middle of the bottom wall, that is in fluid connection to the internal adapter channel (131). In a preferred embodiment, the cross-section of the sidewall of the second opening (112) is annular and the reservoir (115) has a corresponding round cross-section, at least at the reservoir opening. In figure 1, the reservoir (115) in form of a syringe (115a) is connected to the second opening (112) via a lockable LUER fitting with thread (LUER lock).

In one embodiment of the invention, the first and/or the second opening (111, 112) comprise connectors, preferably on, or as part of, the bottom wall (111a, 112a), that enable the connection to the vial (113) or to the reservoir (115), respectively. These connectors may take various forms. In one embodiment, the connectors of the first and/or the second opening (111, 112) are fashioned as LUER fittings. In a specific ambodiment, the first and/or the second opening (111, 112) are fashioned as a male LUER fitting, and the vial (113) and/or the reservoir (115) are fashioned as female LUER fitting. In a more specific embodiment, the first and/or the second opening (111, 112) are fashioned as LUER locks, which are lockable by a half turn of the vial (113) or the reservoir (115), and the vial (113) and/or the reservoir (115) are fashioned as the corresponding LUER fitting with or without threads such that a releasable locked connection is etablished. The first and/or the second opening (111, 112) can comprise the same connector, e.g. a male LUER fittings, or different connectors, e.g. the first opening (111) can comprise a LUER fitting while the second opening (112) can comprise a fitting other than a LUER fitting. In a preferred embodiment, first opening (111) comprises no connector while the second opening (112) comprises a LUER fitting with thread (LUER lock). In the exemplary embodiment shown in figures 2, 4c and 4d, the second opening (112) has a connector (136) in form of a LUER-slip (without thread) at the bottom of the second opening (112) which is able to connect to a reservoir (115).

In one embodiment of the invention, the second opening (112) is equipped with a reservoir locking means for unreleasably connecting the reservoir (115) to the second opening (112). The reservoir locking means of the second opening (112) can have the same properties as described above for the vial locking means (116) of the first opening (111).

According to the invention, the reservoir (115) can be provided in any suitable form or shape known to the skilled person to be suitable for housing an injectable liquid; for example in the form of a flask, a bottle, a tube, or a cylinder.

In one embodiment of the invention, the reservoir (115) comprises a housing (117) and a piston (118) movable relative to and within the housing for aspiring and extruding the injectable liquid, as present for example in a syringe (115a). This embodiment is depicted in figure 1, figure 3, figure 5, and figure 6. In one embodiment, the reservoir (115) has connectors corresponding to the connectors of the second opening (112) as described above. In a specific embodiment, the reservoir (115) is a syringe equipped with a LUER-slip (without a thread) or with a LUER lock (with a thread).

Instead of, or in addition to, the locking means of the second opening (112), the reservoir (115) can also be equipped with locking means for unreleasably connecting the reservoir (115) to the second opening (112). These locking means can have the same form or shape as described above for the locking means of the second opening (112). In a specific embodiment, these locking means are part of the connectors of the second opening (112) and the reservoir (115).

According to the invention, the reservoir (115) exhibits a second longitudinal central axis (B). The second longitudinal central axis (B) reaches from one end of the reservoir (115) that faces away from the adapter (100) to another end of the reservoir (115) that faces in the direction of the adapter (100). In most cases, when the reservoir (115) has a cylindric form, the second longitudinal central axis (B) can be described as being parallel to the sidewall of the housing (117) of the reservoir (115). In most embodiments, the reservoir (115) comprises a closure, like e.g. a plug, a lid, a membrane or any means suitable for closing the reservoir (115) before connecting the reservoir (115) to the adapter (100); preferably the closure is suitable for aseptically closing the reservoir (115). In one embodiment of the invention, the reservoir (115) comprises a lid, preferably a pierceable lid. In a preferred embodiment, the housing (117) of the reservoir (115) comprises a transparent or at least partially transparent outer wall. This is advantageous when the degree of mixture, or, in other words, the mixture's homogeneity, is to be checked during the preparation of the drug formulation.

According to the invention, the first and the second openings (111, 112) are arranged to receive and hold the vial (113) and the reservoir (115), respectively, in a position with the first and the second longitudinal central axes (A, B) positioned at an angle below 75°. In some embodiments, the first and the second longitudinal central axes (A, B) are positioned at an angle below 60°, preferably below 50°, or more preferably below 20°. These smaller angles, with the vial (113) and the reservoir (115) being inclined towards one another, are advantegous in that the adapter (100) can be used to build, or assemble, a drug mixing and injection system (200) that is smaller and more easily manoeuvrable than systems with an angle of 90° or more. The smaller size and the spatial proximity of the vial (113) and the reservoir (115) when fit into the adapter (100) make the drug mixing and injection system (200) equipped with said adapter (100) far more easy-to-handle. This easy operability and advantageous spatial arrangement allows that the reservoir (115) does not have to be removed from the adapter (100), or from the drug mixing and injection system (200), for administration. Instead, both the vial (113) and the reservoir (115) can remain fitted in the adapter (100) during administration; e.g. during intravenous injection.

In one of the preferred embodiments of the invention, the first and the second longitudinal central axes (A, B) are positioned at an angle between 50° and 0°, preferably between 20° and 0°, more preferably between 10° and 0°, or at an angle from 10° to 0°.

In a further preferred embodiment of the invention, the first and the second longitudinal central axes (A, B) are positioned at an angle between 5° and 0°, or at an angle from 5° to 0°. This corresponds to an essentially parallel position of the first and the second longitudinal axes (A, B), and thus of the vial (113) and the reservoir (115). This is even more advantegous as the assembled drug mixing and injection system can be even smaller in size and the respective weights of the vial (113) and reservoir (115) have even less impact on the overall weight distribution of the drug mixing and injection system; thus, the drug mixing and injection system has a better balance and is easier to hold and to handle during preparation of the drug formulation with the injectable liquid, like the mixing steps as well as during administration.

Figure 5 depicts the drug mixing and injection system (200) according to the second aspect of the invention in the exemplary embodiment where the first and the second longitudinal central axes (A and B) of the vial (113) and the reservoir (115), respectively, are positioned at an angle of ca. 0°, or in other words, being parallel to eachother. The openings of the vial (113) and the reservoir (115), which in the shown embodiment is a syringe (115a), both face in the direction of the outlet port (120). In other word, the first and the second opening (110, 120 - not shown in the figure 5) face in the same direction, namely upwards, when the system is held in the normal operational, upright position with the outlet port (122) facing downwards.

In further embodiments, the first and the second central axes (A, B) are positioned at an angle between 75° and 5°, or between 70° and 20°, or between 60° and 30°, or between 50° and 40°.

In a preferred embodiment, the first subunit (110) exhibits not more than two openings for receiving and holding a vial or a reservoir, such as a syringe (115a); in other words, the first subunit (110) does not comprise any further openings that are adapted to receive and hold a vial or a reservoir, such as a syringe (115a). In this embodiment, the first subunit (110) contains only two openings (111, 112), namely, the first opening (111) adapted to receive and hold a vial (113) and the second opening (112) adapted to receive and hold a reservoir (115) comprising an injectable liquid, such as a syringe (115a). Thus, in this embodiment, the drug mixing and injection device (200) according to the second aspect of the invention contains one vial (113) and one reservoir (115) but no further vials or reservoirs. Preferably, the reservoir (115) is prefilled with an injectable liquid (i.e. filled prior to its insertion into its respective opening in the adapter (100)), thereby enabling the drug mixing and injection device (200) to be operable without the need for a separate container in addition to the vial (113) and in addition to the reservoir (115) for providing the injectable liquid, and in particular without the need to have said additional, separate container attached to, or inserted in, the adapter (100) of the drug mixing and injection device (200) during administration. This embodiment of an adapter (100) with only two openings (first and second openings (111, 112)), and optionally its use with a prefilled reservoir (115) comprising the injectable liquid, is advantegous because it keeps the drug mixing and injection device (200) small and light-weight, and it can omit unnecessary use of material (e.g. omitting the need of an additional, separate container). The omission of an additional, separate container, or specifically the omission of a further opening for its insertion in the adapter (100), also yields systems that have a compact shape and beneficial spatial arrangement, and thus are easier to handle - especially single-handedly - than, for instance, injection devices in which said additional, separate container (e.g. a second vial) remains inserted in the adapter.

According to the invention, besides the first subunit (110) which receives and holds the vial (113) and the reservoir (115), the adapter (100) also comprises a second subunit (120), said second subunit (120) comprising a valve (121) and an outlet port (122). The second subunit (120) is the lower of the two subunits when the adapter (100) is held in the normal operational, upright position with the outlet port shoing downwards.

According to the invention, the valve (121) is adapted to switch at least between a first position enabling a first fluid flow between the first opening (111) and the second opening (112), and blocking the fluid connection to the outlet port (122); and a second position enabling a second fluid flow between the second opening (112) and the outlet port (122), and blocking the fluid connection to the first opening (111). In the exemplary embodiments depicted in figures 6a, 6b and 6c, the valve (121) is shown in its first position, while in figures 1 and 6d the valve (121) is shown in its second position. When the valve (121) is in its first position, the injectable liquid can be transfered from the reservoir (115) to the vial (113) and back. In this first position, the drug mixing and injection device (200) comprising the adapter (100) is able to perform the function of preparing an injectable drug formulation by mixing the injectable liquid with the drug product in powder form or liquid in the vial (113). The transfer of the injectable liquid from the reservoir (115) to the vial (113) and vice versa, can be performed several times until the drug is mixed with the injectable liquid to the desired extent, which is typically, a homogeneous solution or, as the case may be, a homogeneous suspension or homogeneous emulsion. When the valve (121) is in its second position, the liquid of the reservoir (115), e.g. the injectable liquid or the prepared injectable drug formulation, can be ejected via the outlet port (122). In this second position, the drug mixing and injection device (200) comprising the adapter (100) can thus be used to perfom an injection, e.g. after attaching an injection needle to the outlet port (122).

The valve (121) can be provided in any form or shape know by the skilled person to be suitable for switching at least between a first and a second position as described above. In one embodiment of the invention, as exemplarily shown in figures 1, 2, 4c and 4d, the valve (121) is a three-way valve having three openings to selectively enable fluid connection between the first opening (111), the second opening (112), and the outlet port (122). The fluid connection is established by means of at least one internal valve channel (131d). In one embodiment, the valve (121) is mechanically operable; for instance, by rotation or by a shift; in other words, the valve (121) can be switched mechanically at least between the above described first position and second position by a rotational movement, or by a shifting, or translational, movement. In case of a rotatable valve (121), the internal valve channels (131d) are typically in the same plane, and the direction of fluid flow in said plane is selected via a rotatable handle. In case of a shiftable valve (121), as exemplarily depicted in figure 2, the internal valve channels (131d) are typically in different planes, for instance, two internal valve channels (131d) in two stacked, parallel planes, and the direction of fluid flow is selected via a shift of the valve's internal channels (131d) between these planes. For instance, when the valve (121) depicted in figure 2 is shifted in the direction perpendicular to the plane of projection of figure 2 (i.e. pushed into or out of said plane), the valve (121) is transfered from one position, e.g. the first position, to the other position, e.g. the second position. As shown in the exemplary embodiment of figure 3, the shiftable valve (121) can comprise a valve knob (121a) which is able to move, or shift, the internal valve channels (131d) within the valve (121) itself such that fluid flow to the internal adapter channel (131) is enabled in the different positions of the valve (121). In one specific embodiment, the valve (121) is adapted to be operated single-handedly. In a preferred embodiment, the shiftable valve (121) is adapted to be operated single-handedly. Valves (121) that can be operated single-handedly can exhibit a valve knob (121a) that is adapted to rest a finger on, such as the ring finger of the human hand (300) or the thumb, e.g. by providing a dedicated site for a finger. The dedicated site for a finger of the valve knob (121a) can have a shape and size that is approximately matching a finger-tip, and is adapted to move the valve by pressing a finger on it. It can, for instance, be plane or have a recess, or a depression. The position of such a valve (121) within the adapter (100) is such that the rest of the adapter can be held comfortably by the other fingers of the same hand whose finger is used for moving the valve knob (121a), as exemplarily shown in figure 7.

In a specific embodiment of the invention, the valve (121) is adapted to switch to a third position blocking any fluid connection between the first opening (111), the second opening (112), and the outlet port (122). This is of advantage when fluid flow of the reservoir (115) to the internal adapter channel (131) is unwanted. This can be the case, for example, while assembling the drug mixing and injection system. In a further specific embodiment, the valve (121) is adapted to switch to a third position blocking any fluid connection between the first opening (111), the second opening (112), and the outlet port (122) in embodiments in which the reservoir (115) has no pierceable lid.

According to the invention, the outlet port (122) is adapted to eject the mixture of the drug and the injectable liquid, e.g. an injectable drug formulation, for instance an injectable drug solution, from the drug mixing and injection system. In alternative embodiments, e.g. when the injectable liquid shall be retrieved from the reservoir (115), the outlet port (112) can be used to eject the injectable liquid from the reservoir. In one of the prefered embodiments of the invention, the outlet port (122) comprises an injection means, or is adapted for attaching an injection means; optionally via connectors such as flexible tubing. The injection means can be provided in any form or shape known to the skilled person to be suitable for injection, like e.g. a needle or an indwelling cannula. For applications other than percutaneous injection, e.g. topical application of a drug formulation to the skin, the outlet port (122) comprises means for application to the skin, e.g. blunt or oblong applicators able to spread the drug formulation on a surface, like spatulas or drippers. As shown exemplarily in figure 2, the outlet port (122) can have a tapered form, like e.g. a LUER fit, able to connect to an injection needle. The presence of the outlet port (122) allows the use of the system as not only a drug mixing device, but as a drug mixing and injection device (200). Thus, the step of disconnecting the reservoir (115) from the adapter (100) in order to subsequently use the reservoir (115) for administration to a subject can be bypassed. This can reduce potential loss of product housed in said reservoir, e.g. the mixture of the drug and the injectable liquid. This also can reduce the risk of contamination of the product inside the reservoir (115) since the reservoir (115) is not in contact with either ambient air and/or the skin of a user both of which can carry microbial and other impurities. Furthermore, not having to disconnect the reservoir (115) from the adapter (100) also can reduce the risk of contamination of the user (e.g. by unintentional spillage of a potentially cytotoxic product) since the outlet opening of the reservoir (115) is not exposed prior to injection. In theory, such spillage and in turn user contamination may also occur via the outlet port (122) of the drug mixing and injection device (200); however, the risk is far lower since the valve can be kept in the first position until the injection means are attached so as to avoid unintentional ejection of product from the outlet port (122).

According to the invention, the adapter (100) comprises at least one internal adapter channel (131) enabling fluid connection between the first opening (111), the second opening (112), and the outlet port (122). The internal adapter channel (131) is a cavity or duct in the inner of the subunits and is adapted to let fluids run through the adapter. It should be understood that, in order to connect the first opening (111), the second opening (112), and the outlet port (122) and enable fluid connection between them, said internal adapter channel (131) is not necessarily shaped as a single linear duct but can be shaped e.g. as a series of branched ducts. The internal adapter channel (131) can comprise at least two parts in the two subunits (110, 120) that are placed such that the endings of the at least two parts of the internal adapter channel (131) are matched and form a fluid connection between the components of the two subunits (110, 120). The matching of the endings of the internal adapter channel (131) is established at least in the activated position of the adapter (100). In the exemplary embodiment shown in figure 2, the internal adapter channel (131) has three parts (131a, 131b, 131c). The first part of the internal adapter channel (131a) is positioned between the valve (121) and the first opening (111), the second part of the internal adapter channel (131b) is positioned between the valve (121) and the second opening (112), and the third part of the internal adapter channel (131c) is positioned between the valve (121) and the outlet port (122). Additionally, figure 2 shows internal valve channels (131d) which enable fluid connection between these three parts of the internal adapter channel (131a, 131b, 131c). The internal valve channels (131d) of the valve (121) can also be seen as being part of the at least one internal adapter channel (131) in that they have the same function, namely enabling fluid connection when operating the drug mixing and injection system (200).

According to the invention, the adapter (100) comprises a first cannula (134) positioned between the valve (121) and the first opening (111), said first cannula (134) being adapted to pierce the lid (114) of the vial (113) and to enable fluid connection between the vial (113) and the at least one internal adapter channel (131). In one embodiment of the invention, the first cannula (134) is positioned between the at least one internal adapter channel (131) and the first opening (111). In some embodiments of the invention, the first cannula (134) is partially introduced in the internal adapter channel (131) and extends in the direction of the first opening (111). In further embodiments, parts of the first cannula (134) enclose parts of the internal adapter channel (131), preferably the parts of the internal adapter channel (131) that extends to the first opening (111).

In one embodiment of the invention, the adapter (100) comprises a second cannula (135) positioned between the valve (121) and the second opening (112), said second cannula (135) being adapted to pierce the lid of the reservoir (115) and to enable a fluid connection between the reservoir (115) and the at least one internal adapter channel (131). In the exemplary embodiment depicted on figure 1, it is shown that the second cannula (135) connects the internal adapter channel (131) in the second subunit (120) with the outlet opening of the syringe (115a). In one of the preferred embodiments of the invention, the pierceable lid of the reservoir (115) is pierced only at the timepoint of activating the adapter shortly prior to use with the help of the second cannula. This embodiment has the advantage that the injectable liquid can be kept sterile more easily during assembly, storage and shipment of the drug mixing and injection system. In one embodiment of the invention, both the vial (113) and the reservoir (115) comprise pierceable lids, and the adapter (100) comprises the first cannula (134) as described above and the second cannula (135) as described above.

The first cannula (134) and the second cannula (135) can have any form or shape known to the skilled person to be suitable for piercing through the pierceable lid(s) of the vial (113) or the reservoir (115), respectively; e.g. a spiky form or a thorn-shaped form. In one embodiment, the first cannula (134) and/or the second cannula (135) have a cylindric shape and at least one spiky end. The first cannula (134) and the second cannula (135) can be made of any material known to the skilled person to be suitable for piercing through the pierceable lid(s) of the vial (113) or the reservoir (115), respectively; i.e. any hard material that gives sufficient resistance to the force needed to pierce through the material of the lid without deformation. These materials can be metal, plastic or ceramics, for instance. The first cannula (134) and the second cannula (135) are positioned as described above or in other ways that enable a fluid flow to the internal adapter channel (131). In a specific embodiment, parts of the first cannula (134) and/or of the second cannula (135) can represent, or form, parts of the internal adapter channel (131).

According to the invention, the first and the second subunits (110, 120) of the adapter (100) are engageable with each other in such a way that the adapter (100) has a resting position in which the first and the second subunits (110, 120) are releasably or unreleasably connected and the first cannula (134) does not pierce through the lid (114) of the vial (113); and an activated position in which the first and the second subunits (110, 120) are shifted towards each other and are in a position in which the first cannula (134) pierces through the lid (114) of the vial (113), thereby enabling fluid connection between the vial (113) and the internal adapter channel (131).

During storage and shipment, the adapter (100) is in its resting position. In this position, the lid (114) of the vial (113) is not pierced and the vial (113) is not open. In the resting position, the first and the second subunits (110, 120) are releasably or unreleasably connected but no fluid connection is yet enabled between the vial (113) and the internal adapter channel (131). In a specific embodiment, when the reservoir (115) comprises a lid, too, also no fluid connection is enabled between the reservoir (115) and the internal adapter channel (131). Thus, during storage and shipment, the drug provided in the vial (113) and/or the injectable liquid provided in the reservoir (115) are kept safe by the adapter (100) and can not be touched, extracted, mixed, diluted or otherwise manipulated. Furthermore, in the resting position, there is a space, or gap, between the first and the second subunits (110, 120) such as exemplarily depicted in figures 4a and 4.

The above has the advantage that the adapter (100) and the drug mixing and injection system (200) comprising the adapter (100) are suitable for long term storage. As the drug is not in contact with any liquid(s) during storage, and thus can, for instance, remain in the favourable dry state, it has a prolonged shelf life. When sterilized as a whole, or when equipped with a sterile vial (113) and a sterile reservoir (115) comprising a lid, the drug mixing and injection system remains sterile within its packaging even under non-sterile storage condiditons.

During mixing of the drug with the injectable liquid, and also during administration of the injectable drug formulation, the adapter (100) is in its activated position. According to the invention, in order to bring the adapter (100) into the activated position, the first and the second subunits (110, 120) are shifted towards each other. In other words, the first and the second subunits (110, 120) are pressed together such that the space, or gap, between the first and the second subunits (110, 120) is reduced. Preferably the space, or gap, is reduced until no space is left between the first and the second subunits (110, 120), as shown, for instance, in figure 4b which depicts an exemplary embodiment of the adapter (100) in the activated position. Further examples of the adapter (100) in the activated position are shown in figures 1, 4d, 5, 6b, 6c and 6d.

According to the invention, in the activated position the first and the second subunits (110, 120) are in a position in which the first cannula (134) pierces through the lid (114) of the vial (113), thereby enabling fluid connection between the vial (113) and the internal adapter channel (131). In some embodiments of the invention, in the activated position, also a fluid connection between reservoir (115) and the at least one internal adapter channel (131) is enabled. This is the case, for instance, when the reservoir (115) has no pierceable lid and/or the fluid connection is already enabled in the resting position. The adapter (100) as exemplarily shown in figure 2 is adapted to receive and hold a vial (113) with a pierceable lid (114) and a reservoir (115) without a pierceable lid. Therefore, this exemplary adapter (100) does not comprise a second cannula (135), but comprises the first cannula (134) positioned between first and second subunit (110, 120). Further, the adapter (100) has no dedicated connector in the first opening (111) but a connector (136) in the second opening (112); here, for instance, a connector (136) in form of a LUER-slip (without thread) at the bottom wall (112a) of the second opening (112).

Alternatively, in some embodiments, the reservoir (115) has a pierceable lid, and in the activated position, the first and the second subunits (110, 120) are in a position in which the second cannula (135) pierces through the lid of the reservoir (115), thereby enabling fluid connection between the reservoir (115) and the internal adapter channel (131). In these embodiments, in the activated position of the adapter (100), the first and the second subunits (110, 120) are shifted towards each other and are in a position in which the first cannula (134) pierces through the lid (114) of the vial (113), thereby enabling fluid connection between the vial (113) and the internal adapter channel (131), and in which the second cannula (135) pierces through the lid of the reservoir (115), thereby enabling fluid connection between the reservoir (115) and the internal adapter channel (131).

In some embodiments of the invention, the first and the second subunits (110, 120) are unreleasably connected when in the activated position. In a more specific embodiment, the first and the second subunits (110, 120) are also unreleasably connected when in the resting position. These embodiments increase the safety and also promote the tamper-proof properties of the drug mixing and injection system (200) as the disassembling of the components of the adapter (100) or the drug mixing and injection system (200) would require brute forces or break the adapter. The possibility to misuse the adapter (100) or the drug mixing and injection system (200) is reduced.

In one embodiment of the invention, the adapter (100) comprises guiding means (132) for guiding the first subunit (110) and the second subunit (120) when shifting them towards each other from the resting position into the activated position as shown in figure 2. The guiding means (132) can have any form known to the skilled person to allow the first and/or the second subunits (110, 120) to slide along one another. In one embodiment, the guiding means (132) is formed as a set of one or more recesses in the first subunit (110) or in the second subunit (120), with the respective other subunit exhibiting the corresponding protrusions so as to guide the sliding movement of the two subunits towards eachother. In one embodiment of the invention, the guiding means (132) are suitable for guiding the first and the second subunits (110, 120) during the assembly of the adapter (100) and/or during setting the resting position of the adapter (100).

In one embodiment of the invention, the first or the second subunit (110, 120) comprise at least one subunit locking means (133) for the engagement, or connection, of the first subunit (110) with the second subunit (120) in such a manner that the two subunits (110, 120) do not disconnect easily and/or unwantedly; e.g. preventing the two subunits (110, 120) from sliding apart within the packaging during shipment and/or storage or upon unpacking and use of the adapter (100). In one embodiment, which is exemplarily shown in figure 2, the subunit locking means (133) is formed as a set of one or more protrusions on the guiding means (132) and corresponding subunit retaining grooves (133b) positioned inside the first and the second subunits (110, 120). Alternatively, the subunit locking means (133) can be formed as a ribbon positioned outside the first and the second subunits (110, 120), and connecting the two subunits (110, 120).

In a more specific embodiment, the first and/or the second subunits (110, 120) comprise at least two subunit locking means (133) for the engagement of the first subunit (110) with the second subunit (120). More specifically, one of the at least two subunit locking means (133) is engaged in the resting position, and another one of the at least two subunit locking means (133) is engaged in the activated position. Alternatively, both of the at least two subunit locking means (133) can be engaged in the resting position and in the activated position.

In a further specific embodiment of the invention, the subunit locking means (133), or at least one of the at least two subunit locking means (133), is formed as at least one subunit retaining hook (133a), as exemplarily depicted in figures 2, 4c and 4d. The at least one subunit retaining hook (133a) can be comprised in the first or in the second subunit (110, 120). In the respective other subunit, the subunit retaining hook (133a) finds corresponding means for hooking into and thereby locking the first and the second subunits (110, 120) together. Alternatively, or in addition to the at least one subunit retaining hook (133a), the subunit locking means (133), or at least one of the at least two subunit locking means (133), may also be formed as at least one rounded, convex knob with at least one corresponding rounded concave subunit retaining groove (133b).

Exemplary figure 4 shows a subunit locking means (133) in form of at least one subunit retaining hook (133a) provided on the guidings means (132) of the second subunit (120). When the adapter (100) is in its resting position, there is a space, or gap, between the first and the second subunits (110, 120), and the subunit retaining hook (133a) interacts with a first corresponding subunit retaining groove (133b) provided in the guiding means (132) of the first subunit (110) (figure 4c). This initial connection of the at least one subunit retaining hook (133a) with its first corresponding subunit retaining groove (133b) is advantageous in that it prevents an unwanted disconnection of the the first and the second subunits (110, 120) as described above. When the adapter (100) is shifted to its activated position, this space, or gap, between the first and the second subunits (110, 120) is reduced, optionally closed; and the subunit retaining hook (133a) interacts with a second corresponding subunit retaining groove (133b) (figure 4d). In the exemplary embodiment depicted in figure 4, the second subunit retaining groove (133b) is positioned above the first subunit retaining groove (133b) when the adapter (100) is held in the upright position, and at such a distance that, when shifting the adapter (100) from its resting into its activated position, the first and the second subunits (110, 120) are connected such that no space is left between the two subunits (110, 120).

As can also be seen from exemplary figures 4c and 4d, the first and/or the second subunits (110, 120) comprise not only one but two subunit locking means (133) for the engagement of the first subunit (110) with the second subunit (120). Specifically, there is depicted a first set of a subunit retaining hook (133a) with its corresponding first and second subunit retaining grooves (133b) on the left-hand side of the adapter (100), facing towards the first opening (111); as well as a second set of a subunit retaining hook (133a) with its corresponding first and second subunit retaining grooves (133b), on the right-hand side of the adapter (100), facing towards the second opening (112). When in resting position, both subunit retaining hooks (133a) interact with their corresponding first subunit retaining groove (133b); thus preventing an unwanted sliding apart of the first and the second subunits (110, 120) even better. When the adapter (100) is shifted from its resting position to its activated position, both subunit retaining hooks (133a) then interact with their corresponding second subunit retaining grooves (133b).

In one embodiment of the invention, the subunit locking means (133) is adapted to unreleasably lock the first and the second subunits (110, 120) of the adapter (100) in the resting position and/or in the activated position with the provision that, in case of an unreleasable lock of the adapter (100) in the resting position, said lock still allows the transfer of the adapter (100) to the activated position. Depending on the shapes and/or material properties (e.g. hardness) chosen, the subunit locking means (133) in form of subunit retaining hook (133a) and corresponsing first and second subunit retaining grooves (133b), as shown in figures 2, 4c and 4d, enables unreleasable connections of the first and the second subunits (110, 120) in the resting as well as in the activated position. For instance, the sloped form of the subunit retaining hook (133a) can be shaped as a barbed hook, thereby rendering the connection between a thus shaped subunit retaining hook (133a) and its corresponding subunit retaining groove(s) (133b) unreleasable.

Alternatively, or in addition thereto, the subunit locking means (133) can have other forms, and/or can be made of any material known to the skilled person whose properties (e.g. hardness, surface adhesion, etc.) allow for the unreleasable connection of the first and the second subunits (110, 120) while still allowing the transfer from the resting into the activated position of the adapter (100) at the same time.

Unreleasably locking the first and the second subunits (110, 120) of the adapter (100) in the resting position and/or in the activated position is advantageous not only because it prevents the two subunits (110, 120) from disconnection (e.g. sliding apart), but also because it renders the drug mixing and injection device (200) less susceptible for tampering and/or contamination (e.g. by microbes). As mentioned above, the disassembling of the components of the adapter (100) or of the drug mixing and injection system (200) would require brute forces or break the adapter (100), thus reducing the possibility to misuse the adapter (100) or the drug mixing and injection system (200).

In one embodiment of the invention, the first and the second subunits (110, 120) are adapted such that they can be shifted towards each other single-handedly. Specifically, the activated position of the adapter (100) can be set, or achieved, single-handedly. In a specific embodiment, the adapter (100) comprises at least one dedicated site for resting a finger (123) used when bringing the adapter (100) from the resting position into the activated position. In a further specific embodiment, the second subunit (120) comprises at least one dedicated site for resting a finger (123) when bringing the adapter (100) from the resting position into the activated position. This facilitates holding and using the adapter (100) single-handedly as it limits the risk of the finger(s) slipping. The dedicated site for resting a finger (123) can be, for instance, an approximately finger-tip sized recess, or depression e.g. a wing-shaped form, or curve, placed at the lower end of the second subunit (120) such as to comfortably rest one or two fingers in the concave part(s) of said form. Preferably, the dedicated site (123) is positioned at the lower part, or lower end, of the second subunit (120) that is facing downwards when the adapter (100) or the drug mixing and injection system is held in the normal operational, upright position with the outlet port (122) showing downwards. In figures 2, 3b and 7, the decicated site(s) for resting a finger (123) are exemplarily shown. The decicated site (123) is designed as protrusions in wing-shaped form and is positioned at the lower part of the second subunit (120). In the specific embodiment depicted in these figures, the user can rest one or two fingers at the decicated site (123); optionally, one finger can be the thumb of the user. In figure 7, the handling of the drug mixing and injection system (200) is shown. The system is held by positioning two fingers, as examplarly shown the forefinger and the middle finger of a human hand (300), on the decicated site for resting a finger (123) and the thumb is positioned on the vial bottom (113a). The embodiment of the drug mixing and injection system (200) comprising a decicated site for resting a finger (123) designed as protrusions in wing-shaped form, and a vial bottom (113a) oriented in a plane approximately parallel to the one of the fingers resting at said decicated site for resting a finger (123), is advantageous when the system is used single-handedly since slipping of the finger(s) can be omitted.

In one embodiment of the invention, the adapter (100), including the at least one internal adapter channel (131), is sterilizable, preferably with a sterilizing gas such as ethylenoxide and/or with gamma irradiation, more preferably with ethylenoxide. In one of the preferred embodiments, the adapter (100) is sterilizable with a sterilizable gas such as ethylenoxide within its primary packaging; for instance, in cases where the adapter (100) is sold separately and not as part of the inventive drug mixing and injection system. In some embodiments, the adapter (100) is sterilizable with a sterilizable gas and comprises at least one dedicated sterilizing path for the inlet of the sterilizable gas into the valve (121). These one or more dedicated sterilizing paths lead from the outside of the adapter (100) to the inner parts of the valve (121) and to the internal valve channels (131d) which are able to establish fluid connection and which, in the time of sterilization, do not have direct access to any of the three openings of the adapter (first opening (111), second opening (112), and outlet port (122)). The at least one dedicated sterilizing path is positioned such that it has no fluid connection to the internal adapter channels (131) and is not in contact with any liquid, especially not with the injectable liquid or with the drug formulation.

In one embodiment of the invention, the vial (113) comprises at least one drug. The at least one drug can be provided in powder form or as a liquid. In a specific embodiment of the invention, the at least one drug is lyophilized; for instance, by a freeze-drying method. In another specific embodiment, the liquid drug is concentrated and/or solubilised.

In one embodiment of the invention, the first subunit (110) is equipped with a gas-permeable and liquid-impermeable filter, said filter being configured to compensate and/or equilibrate pressure. The filter can have any form or shape and be made of any material and compositions known to the skilled person to be suitable for compensating and/or equilibrating pressure.

In one embodiment of the invention, the adapter (100) is preassembled prior to use. When the adapter (100) is preassembled, the first and the second subunits (110, 120) are releasable or unreleasable connected. In some embodiments, the adapter (100) is in its resting position when preassembled. The assembly of the drug mixing and injection system follows the preassembly of the adapter (100) either directly (typically at the manufacturer's site), or after a storage and/or shipment interval (at the manufacturer's site or at the users' site). In the preassembled state of the adapter (100), the valve (121) is in its first, second, or third position. In one embodiment of the invention, the adapter (100) is sterile packaged, or in other words packed under sterile conditions or sterilized after packaging.

In one embodiment of the invention, the adapter (100) is intended for single-use. The single-use adapter can be discarded after use together with the vial (113) and the reservoir (115).

In a second aspect, the invention relates to a drug mixing and injection system (200) comprising the adapter (100) of the first aspect of the invention, a vial (113) and a reservoir (115) with injectable liquid. According to this second aspect, the drug mixing and injection system (200) comprises the adapter (100) according to the first aspect of the invention as described above, a vial (113) comprising a pierceable lid (114) and exhibiting a first longitudinal central axis (A), and a reservoir (115) comprising an injectable liquid and exhibiting a second longitudinal central axis (B).

An example of the components of the drug mixing and injection system (200) is shown from various perspectives in the embodiment of figure 3. Figure 3a (front view) and 3b (tilted from below) show the adapter (100) in the activated position with the first and the second subunits (110, 120), the valve (121) with the valve knob (121a), the outlet port (122) and a decicated site for resting a finger (123). Further, figure 3a and 3b show the vial (113), the reservoir (115) in form of a syringe (115a) with the housing of the reservoir (117) and the piston of the reservoir (118). Some of the compontents can be seen in the bottom view of figure 3c, especially, the second subunit (120), the decicated site for resting a finger (123), the outlet port (122) and the valve knob (121a).

The drug mixing and injection system (200) according to the second aspect of the invention can be used for the preparation of a drug formulation and the injection of it into humans, typically percutaneously. Preparation of a drug formulation can comprise diluting or mixing a drug with a liquid, typically an injectable liquid. The resulting drug formulation can be a solution (also referred to as a molecular dispersion; typically below 1 nm particle size), a colloidal solution (also referred to as a colloidal dispersion, typically 1-100 nm particle size), or a suspension (typically above 100 nm particle size), or any other mixture achieved by adding an injectable liquid to a drug and mixing it with the drug mixing and injection system (200). Alternatively, the drug mixing and injection system (200) can be used for administration of drug formulations onto the skin of the body. Additionally, the drug mixing and injection system (200) can also be used for injection of a drug formulation into a test vial and/or test device in research settings or the like (e.g. a spectrometer).

In one embodiment of the invention, the drug mixing and injection system (200) is preassembled prior to use. In the preassembled state, all parts of the drug mixing and injection system (200), namely the adapter (100), the vial (113), and the reservoir (115) are set together: the vial (113) is connected to the adapter (100) via the first opening (111) of the first subunit (110); the reservoir (115) is connected to the adapter (100) via the second opening (112). When the adapter (100) exhibits a vial locking means (116) or a reservoir locking means, the vial (113) or the reservoir (115), respectively, are connected to the first or second opening (111, 112) until the unreleasably state of the connection is established. The preassembled drug mixing and injection device (200) has the advantage that the drug as well as the injectable liquid are kept sterile throughout the whole preparation step. The preassembled drug mixing and injection system (200) is thus in a state that is ready to use as there is no need for an additional time-consuming assembling step of connecting the vial (113) and the reservoir (115) to the adapter (100). This increases the ease-of-use properties of the drug mixing and injection system (200) and promotes sterility.

In one embodiment of the invention, the drug mixing and injection system (200) is sterilizable in the preassembled state. In some embodiments, the drug mixing and injection system (200) is sterilizable, preferably with a sterilizing gas such as ethylenoxide and/or gamma irradiation, more preferably with ethylenoxide. In some embodiments, the sterilisation can take place with the assembled drug mixing and injection system (200) within a package. The package material can be any suitable material that is gas permeable for a sterilizing gas, like ethylenoxide, but is unpermeable for contaminating microorganism, like bacteria or viruses.

In one embodiment of the invention, the drug mixing and injection system (200) is sterile packaged, or in other words, it is packed under sterile conditions and/or sterilized after packaging. A sterile packaged system is provided by the sterilized preassembled system in a package as described above. Alternatively, the sterile packaged system can be provided by a system assembled under sterile or aseptic conditions, e.g. under a clean workbench and packaged under sterile conditions.

In one embodiment, the adapter (100) of the drug mixing and injection system (200) is packaged in its resting position for shipping, delivery and storage, optionally, with the first and the second subunits (110, 120) of the adapter (100) being unreleasably connected.

In one embodiment of the drug mixing and injection system (200), the reservoir (115) comprises a lid, optionally a pierceable lid.

In one embodiment of the drug mixing and injection system (200), both the vial (113) and the reservoir (115) comprise pierceable lids and the adapter (100) comprises a first cannula (134) and a second cannula (135) as described above for the adapter (100) according to the first aspect of the invention.

In one embodiment, the drug mixing and injection system (200) is intended for single-use. The single-use drug mixing and injection system can be discarded after use.

In a third aspect, the invention relates to a method of preparing an injectable drug formulation comprising the following subsequent steps:
a) providing a drug mixing and injection system (200) as described above;
b) activating the drug mixing and injection system (200) such that the adapter (100) is transferred from the resting position into the activated position;
c) checking as to whether the valve (121) of the adapter (100) of the drug mixing and injection system (200) as provided under step a) is in its first position and if not, bringing the valve (121) into the first position;
d) transferring the injectable liquid from the reservoir (115) to the vial (113) resulting in a mixture of the drug and the injectable liquid;
e) transferring the resulting mixture of step d) from the vial (113) back into the reservoir (115);
f) checking the resulting mixture of step e) as to whether the drug is mixed with the injectable liquid to a desired extent and if not, repeating steps d) and e), and/or shaking the drug mixing and injection system (200);
g) once in the resulting mixture the drug is mixed with the injectable liquid to the desired extent, and transferred to the reservoir (115) according to step e), bringing the valve (121) into the second position, enabling the second fluid flow;
h) transferring the resulting mixture from the reservoir (115) to the outlet port (122).

The method of preparing a drug formulation is suitable for the preparation of drugs intended for injection into a human or animal body (typically percutaneous), or for administration onto the skin. These drugs are stored in dry or concentrated state and need to be dissolved or diluted prior to use with an injectable liquid. As described above, the resulting injectable drug formulation can be a solution, a colloid, a suspension, or any other mixture achieved by adding an injectable liquid to a drug and mixing it with the drug mixing and injection system (200).

In one embodiment of the method, the step a) of providing a drug mixing and injection system (200), comprises the steps of:
- providing an adapter (100) as described above under the first aspect of the invention,
- connecting to the first opening (111) of the adapter (100) a vial (113) comprising a pierceable lid (114) and exhibiting a first longitudinal central axis (A), and
- connecting to the second opening (112) of the adapter (100) a reservoir (115) comprising an injectable liquid and exhibiting a second longitudinal axis (B).

When connecting the reservoir (115) to the second opening (112) of the adapter (100), the closure of the reservoir (115) is removed before attaching the reservoir. In other embodiments, the closure is removed during the attachment, e.g. by the impact of the connector (136). In yet another embodiment, when the reservoir comprises a piercable lid, the closure is removed or opened, respectively, by the second cannula (135) when the adapter (100) is in the activated position.

In one of the preferred embodiments, step a) further comprises the step of checking as to whether the adapter (100) is in its resting position, and if not bringing the adapter (100) in the resting position. The exemplary figure 6a shows the drug mixing and injection system (200) with the adapater (100) in the resting position and the valve (121) in its first position as provided in some embodiments of step a). The situation after step c) is shown in exemplary figure 6b in which the adapater (100) is in the activated position and the valve (121) is in its first position in order to enable fluid flow between the reservoir (115) and the vial (113). Exemplary figure 6c illustrates the situation after step d); the adapater (100) is in the activated position, the piston of the syringe (118) is pressed down and the injectable liquid is expelled from the reservoir (115) and transfered to the vial (113). Exemplary figure 6d shows the situation after step g). The adapater (100) is in the activated position and the resulting mixture, the injectable drug formulation, is transferred to the reservoir (115) and ready to use. The valve (121) is switched to its second position enabling fluid flow between the second opening (112) and the outlet port (122).

In one embodiment of the method, the adapter (100) is provided preassembled. For preassembling the adapter (100), the first and the second subunits (110, 120) are releasably or unreleasably connected, optionally with the help of the guiding means (132) and/or the subunit locking means (133).

In one embodiment of the method, step a) of providing a drug mixing and injection system (200) is carried out under sterile conditions. In a further embodiment, the preassembling of the adapter (100) is carried out under sterile conditions.

In one embodiment of the method, step a) of providing a drug mixing and injection system (200) further comprises the step of sterilizing the drug mixing and injection system (200), preferably with a sterilizing gas such as ethylenoxide and/or gamma irradiation, more preferably with ethylenoxide. In a specific embodiment, step a) further comprises the step of packaging the assembled drug mixing and injection system (200). The sterilisation step is carried out before or after the packaging step. When carrying out the sterilsation step after packaging the drug mixing and injection system (200), the package is made of any material known to the skilled person to allow sterilisation of the packaged drug mixing and injection system (200), e.g. any material which exhibits pores allowing gas molecules like ethylenoxide molecules to permeate, but which is unpermeable for contaminating microorganisms. In some embodiments, the valve (121) is set to ist first position before the sterilizing step. sterilizing In some embodiments, when carrying out the sterilsation step after packaging, the adapter (100) comprises internal valve paths and the valve (121) is in its first position.

In one embodiment of the method, the valve (121) of the adapter (100) of the drug mixing and injection system (200) as provided under step a) is in its first position. In the specific embodiment wherein the reservoir (115) comprises a pierceable lid, the fluid flow is enabled after step b) of the above method. In other embodiments, wherein the reservoir (115) comprises no lid, or a lid that is opened during the connection of the reservoir (115) to the adapter (100), the fluid flow between second opening (112) and the at lest one internal adapter channel (131) is already enabled before step b) of the above method.

In some embodiments of the method, the valve (121) of the adapter (100) of the drug mixing and injection system (200) as provided under step a) is in its third position. In specific embodiments, the valve (121) is provided in its third position, and the reservoir (115) comprises no lid, or a lid that is opened during the connection of the reservoir (115) to the adapter (100). In these embodiments, no fluid flow is enabled between the second opening (112) and the first opening (111) or the outlet port (122) until step b) is carried out.

In some embodiments of the method, the drug mixing and injection system (200) provided under step a) is adapted such that it is feasible to carry out at least the activating step b) single-handedly; i.e. using just one hand. This can be achieved, or facilitated, by an embodiment (as exemplarily depicted in figure 7), wherein the second subunit (120) of the adapter (100) has a dedicated site for a finger (123) and the vial has a vial bottom (113a), and wherein, preferably, these two are positioned in approximately parallel planes such as two allow the human hand (300) to hold and safely "clamp" the drug mixing and injection system (200) between the thumb and at least one, preferably two, finger(s) without slipping; at the very least during the activating step b). In a preferred embodiment of step b) of the method, as shown in figure 7, two fingers, prefereably forefinger and middle finger, of the human hand (300) are positioned on the dedicated site for resting a finger (123) of the second subunit (120), and the thumb, or one finger or two fingers, of the human hand (300) are positioned on the vial bottom (113a).

When performing step f) of the method, the resulting mixture is typically checked in order to ensure that the drug is mixed with the injectable liquid to the desired extent, which is typically, a homogeneous solution or, as the case may be, a homogeneous suspension or homogeneous emulsion. In one embodiment, the checking is done by visually monitoring the mixture with bare eyes through the transparent or partially transparent outer wall of the housing (117) of the reservoir (115). The desired extent of the mixing depends on the properties of the drug and the intended use of the method. In some cases, the drug and the injectable liquid are mixed but the drug product is not, or only partially, dissolved in the liquid, e.g. in cases of preparing suspensions, colloidal solutions, emulsions, or the like. In many cases, the desired extent is complete dissolution of the drug product in the injectable liquid, as typically recognised when no residues or undissolved particles can be seen with bare eyes during the checking of step f). In case the extent of mixing or dissolving is not as desired, e.g. as necessary for the use, steps d) and e) of the method can be repeated or the drug mixing and injection system (200) can be shaked, or both. During steps d) through f), the valve is in the first position. This makes sure that the mixture of the drug and the injectable liquid is not transferred to the outlet port (122) and ejected.

In some embodiments of the method, the method further comprises the step of
i) disposing the drug mixing and injection system (200).

In some embodiments of the method, the steps are performed consecutively from step a) to step h), or from step a) to step i).

In summary, the present invention provides drug mixing and injection systems, or devices, that reduce the contamination risks as well as the number of handling steps during the preparation of drug formulations to a minimum, and thus enable, or facilitate, aseptic preparation of a drug formulation prior to administration even by lay people.

The inventive drug mixing and injection systems, or devices, enable sterile handling throughout the whole drug formulation preparation and administration process, including transfer of the injectable liquid into the drug-containing vial (113), mixing drug and the injectable liquid, and transfer of the resulting injectable drug formulation back to the reservoir (115) for the administration of the injectable drug formulation. The device is provided sterile as a preassembled drug mixing and injection system and the system remains assembled, and thus closed and sterile, with no openings to the environment the would allow contact to contaminating microorganisms, throughout the whole process.

At the same time, the inventive drug mixing and injection system enables the reconstitution of a drug without losses. The amounts of the drug and the injectable drug formulation can, for instance, be determined by the manufacturer by providing a drug mixing and injection system with all necceassary components and in the necessary amount. Thus, there is no need, that the user - be it a patient or a medically trained person - measure and check the components.

Furthermore, the inventive drug mixing and injection systems, or devices, are easily manageable, e.g. by using one single hand, both during the preparation of the injectable drug formulation as well as its administration. This facilitates easier self-administration for patients and/or safer handling for medically trained staff.

Furthermore, the inventive drug mixing and injection systems, or devices, can increase the safety and promote the tamper-proof properties of such systems since the system's components can be adapted in such a way that their disassembly would require brute force or break the device; hence, the possibility to misuse of both the system itself and/or the drug contained in it is reduced.

Furthermore, the inventive drug mixing and injection systems are material-saving as they use a reduced number of vials which reduces the consumption of disposable material.

## Claims

1. An adapter (100) for a drug mixing and injection system (200) comprising
- a first subunit (110) comprising
- a first opening (111) adapted to hold a vial (113), said vial (113) comprising a pierceable lid (114), and exhibiting a first longitudinal central axis, and
- a second opening (112) adapted to hold a reservoir (115) comprising an injectable liquid, and exhibiting a second longitudinal central axis,
wherein the first and the second openings (111, 112) are arranged to hold the vial (113) and the reservoir (115) in a position with the first and the second longitudinal central axes positioned at an angle below 75°;
- a second subunit (120) comprising an outlet port (122); and
- a first cannula (134) adapted to pierce the lid (114) of the vial (113);
wherein the first and the second subunits (110, 120) are engageable with each other, in such a way that the adapter (100) has
- a resting position in which the first and the second subunits (110, 120) are releasably or unreleasably connected and the first cannula (134) does not pierce through the lid (114) of the vial (113); and
- an activated position in which the first and the second subunits (110, 120) are shifted towards each other and are in a position in which the first cannula (134) pierces through the lid (114) of the vial (113),
**characterized in that**:
- the adapter (100) comprises at least one internal adapter channel (131) enabling fluid connection between the first opening (111), the second opening (112), and the outlet port (122);
- the second subunit (120) further comprises a valve (121) adapted to switch at least between
- a first position enabling a first fluid flow between the first opening (111) and the second opening (112), and blocking the fluid connection to the outlet port (122), and
- a second position enabling a second fluid flow between the second opening (112) and the outlet port (122), and blocking the fluid connection to the first opening (111);
- the first cannula (134) is positioned between the valve (121) and the first opening (111) and is further adapted to enable fluid connection between the vial (113) and the at least one internal adapter channel (131) upon piercing the lid (114) of the vial (113), and
and further **characterized in that**:
- the reservoir (115) is provided in the form of a syringe (115a); and
- the outlet port (122) is adapted such that it can be used to perform an injection after attaching an injection needle to the outlet port (122).

2. The adapter (100) according to claim 1, wherein the adapter (100) comprises guiding means (132) for guiding the first subunit (110) and the second subunit (120) when shifting them towards each other from the resting position into the activated position and/or wherein the first or the second subunit (110, 120) comprise at least one subunit locking means (133) for the engagement of the first subunit (110) with the second subunit (120).

3. The adapter (100) according to claims 1 or 2, wherein in the resting position the first and the second subunits (110, 120) are unreleasably connected, or wherein in the resting position and in the activated position the first and the second subunits (110, 120) are unreleasably connected.

4. The adapter (100) according to any of the previous claims, wherein the first and the second longitudinal central axes are positioned at an angle between 50° and 0°, preferably between 20° and 0°, more preferably between 10° and 0°.

5. The adapter (100) according to any of the previous claims, wherein the first opening (111) is equipped with a vial locking means (116) for unreleasably connecting the vial (113) to the first opening (111).

6. The adapter (100) according to any of the previous claims, wherein the adapter, preferably the second subunit, further comprises at least one dedicated site for resting a finger when bringing the adapter (100) from the resting position into the activated position.

7. The adapter (100) according to any of the previous claims, wherein the adapter (100) is sterilizable.

8. The adapter (100) according to any of the previous claims, wherein the adapter (100) comprises a second cannula (135) positioned between the valve (121) and the second opening (112), said second cannula (135) being adapted to pierce the lid of the reservoir (115) and to enable a fluid connection between the reservoir (115) and the at least one internal adapter channel (131).

9. The adapter (100) according to any of the previous claims, wherein the first subunit (110) has a gas-permeable and liquid-impermeable filter, said filter being configured to compensate and/or equilibrate pressure.

10. A drug mixing and injection system (200) comprising
the adapter (100) according to any of the claims 1 to 9,
a vial (113) comprising a pierceable lid (114) and exhibiting a first longitudinal central axis, and
a reservoir (115) comprising an injectable liquid and exhibiting a second longitudinal central axis.

11. The drug mixing and injection system (200) according to claim 10, wherein the injection system is preassembled prior to use.

12. The drug mixing and injection system (200) according to claim 11, wherein the injection system is sterilizable in the preassembled state.

13. A method of preparing an injectable drug formulation comprising the following subsequent steps:
a) providing a drug mixing and injection system (200) according to any one of the claims 10 to 12;
b) activating the drug mixing and injection system (200) such that the adapter (100) is transferred from the resting position into the activated position;
c) checking as to whether the valve (121) of the adapter (100) of the drug mixing and injection system (200) as provided under step a) is in its first position and if not, bringing the valve (121) into the first position;
d) transferring the injectable liquid from the reservoir (115) to the vial (113) resulting in a mixture of the drug and the injectable liquid;
e) transferring the resulting mixture of step d) from the vial (113) back into the reservoir (115);
f) checking the resulting mixture of step e) as to whether the drug is mixed with the injectable liquid to a desired extent homogeneity and if not, repeating steps d) and e), and/or shaking the drug mixing and injection system (200);
g) once in the resulting mixture the drug is mixed with the injectable liquid to the desired extent, and transferred to the reservoir (115) according to step e), bringing the valve (121) into the second position, enabling the second fluid flow;
h) transferring the resulting mixture from the reservoir (115) to the outlet port (122).

14. The method according to claim 13, wherein step a) comprises the steps
- providing an adapter (100) according to any one of the claims 1 to 9,
- connecting to the first opening (111) of the adapter (100) a vial (113) comprising a lid (114) and exhibiting a first longitudinal central axis,
- connecting to the second opening (112) of the adapter (100) a reservoir (115) comprising an injectable liquid and exhibiting a second longitudinal axis, and
- optionally checking as to whether the adapter (100) is in its resting position, and if not bringing the adapter (100) in the resting position.

15. The method according to claims 13 or 14, wherein the drug mixing and injection system (200) provided under step a) is adapted such that it is feasible to carry out the activating step b) single-handedly.

## Patentansprüche

1. Adapter (100) für ein Arzneimittelmisch- und -injektionssystem (200), umfassend
- eine erste Untereinheit (110), umfassend
- eine erste Öffnung (111), die dazu angepasst ist, ein Fläschchen (113) zu halten, wobei das Fläschchen (113) einen durchstechbaren Deckel (114) umfasst und eine erste zentrale Längsachse aufweist, und
- eine zweite Öffnung (112), die dazu angepasst ist, einen Behälter (115) zu halten, der eine injizierbare Flüssigkeit umfasst und eine zweite zentrale Längsachse aufweist,
wobei die erste und die zweite Öffnung (111, 112) dazu angeordnet sind, das Fläschchen (113) und den Behälter (115) in einer Position zu halten, in der die erste und die zweite zentrale Längsachse in einem Winkel unter 75° positioniert sind;
- eine zweite Untereinheit (120), die eine Auslassöffnung (122) umfasst; und
- eine erste Kanüle (134), die dazu angepasst ist, den Deckel (114) des Fläschchens (113) zu durchstechen;
wobei die erste und die zweite Untereinheit (110, 120) derart miteinander in Eingriff bringbar sind, dass der Adapter (100)
- eine Ruheposition hat, in der die erste und die zweite Untereinheit (110, 120) lösbar oder unlösbar verbunden sind und die erste Kanüle (134) den Deckel (114) des Fläschchens (113) nicht durchsticht; und
- eine aktivierte Position hat, in der die erste und die zweite Untereinheit (110, 120) aufeinander zu verschoben und in einer Position sind, in der die erste Kanüle (134) den Deckel (114) des Fläschchens (113) durchsticht,
**dadurch gekennzeichnet, dass**:
- der Adapter (100) mindestens einen inneren Adapterkanal (131) umfasst, der die Fluidverbindung zwischen der ersten Öffnung (111), der zweiten Öffnung (112) und der Auslassöffnung (122) ermöglicht;
- die zweite Untereinheit (120) ferner ein Ventil (121) umfasst, das dazu angepasst ist, mindestens zu wechseln zwischen
- einer ersten Position, die eine erste Fluidströmung zwischen der ersten Öffnung (111) und der zweiten Öffnung (112) ermöglicht und die Fluidverbindung zu der Auslassöffnung (122) blockiert, und
- einer zweiten Position, die eine zweite Fluidströmung zwischen der zweiten Öffnung (112) und der Auslassöffnung (122) ermöglicht und die Fluidverbindung zu der ersten Öffnung (111) blockiert;
- die zweite Kanüle (134) zwischen dem Ventil (121) und der ersten Öffnung (111) positioniert und ferner dazu angepasst ist, die Fluidverbindung zwischen dem Fläschchen (113) und dem mindestens einen inneren Adapterkanal (131) beim Durchstechen des Deckels (114) des Fläschchens (113) zu ermöglichen, und
und ferner **dadurch gekennzeichnet, dass**:
- der Behälter (115) in Form einer Spritze (115a) bereitgestellt ist; und
- die Auslassöffnung (122) derart angepasst ist, dass sie verwendet werden kann, um nach dem Anbringen einer Injektionsnadel an der Auslassöffnung (122) eine Injektion durchzuführen.

2. Adapter (100) nach Anspruch 1, wobei der Adapter (100) Führungsmittel (132) zum Führen der ersten Untereinheit (110) und der zweiten Untereinheit (120) beim Aufeinanderzuverschieben aus der Ruheposition in die aktivierte Position umfasst und/oder wobei die erste oder die zweite Untereinheit (110, 120) mindestens ein Untereinheitsverriegelungsmittel (133) zum Ineingriffnehmen der zweiten Untereinheit (120) durch die erste Untereinheit (110) umfasst.

3. Adapter (100) nach Anspruch 1 oder 2, wobei in der Ruheposition die erste und die zweite Untereinheit (110, 120) unlösbar verbunden sind, oder wobei in der Ruheposition und in der aktivierten Position die erste und die zweite Untereinheit (110, 120) unlösbar verbunden sind.

4. Adapter (100) nach einem der vorhergehenden Ansprüche, wobei die erste und die zweite zentrale Längsachse in einem Winkel zwischen 50° und 0°, vorzugsweise zwischen 20° und 0°, noch bevorzugter zwischen 10° und 0° positioniert sind.

5. Adapter (100) nach einem der vorhergehenden Ansprüche, wobei die erste Öffnung (111) mit einem Fläschchenverriegelungsmittel (116) zum unlösbaren Verbinden des Fläschchens (113) mit der ersten Öffnung (111) ausgestattet ist.

6. Adapter (100) nach einem der vorhergehenden Ansprüche, wobei der Adapter, vorzugsweise die zweite Untereinheit, ferner mindestens eine spezielle Stelle zum Ablegen eines Fingers umfasst, wenn der Adapter (100) aus der Ruheposition in die aktivierte Position gebracht wird.

7. Adapter (100) nach einem der vorhergehenden Ansprüche, wobei der Adapter (100) sterilisierbar ist.

8. Adapter (100) nach einem der vorhergehenden Ansprüche, wobei der Adapter (100) eine zweite Kanüle (135) umfasst, die zwischen dem Ventil (121) und der zweiten Öffnung (112) positioniert ist, wobei die zweite Kanüle (135) dazu angepasst ist, den Deckel des Behälters (115) zu durchstechen und eine Fluidverbindung zwischen dem Behälter (115) und dem mindestens einen inneren Adapterkanal (131) zu ermöglichen.

9. Adapter (100) nach einem der vorhergehenden Ansprüche, wobei die erste Untereinheit (110) einen gaspermeablen und flüssigkeitsimpermeablen Filter hat, wobei der Filter dazu ausgelegt ist, Druck zu kompensieren und/oder zu äquilibrieren.

10. Arzneimittelmisch- und -injektionssystem (200), umfassend
den Adapter (100) nach einem der Ansprüche 1 bis 9,
ein Fläschchen (113), das einen durchstechbaren Deckel (114) umfasst und eine erste zentrale Längsachse aufweist, und
einen Behälter (115), der eine injizierbare Flüssigkeit umfasst und eine zweite zentrale Längsachse aufweist.

11. Arzneimittelmisch- und -injektionssystem (200) nach Anspruch 10, wobei das Injektionssytem vor der Verwendung vormontiert wird.

12. Arzneimittelmisch- und -injektionssystem (200) nach Anspruch 11, wobei das Injektionssystem im vormontierten Zustand sterilisierbar ist.

13. Verfahren zur Herstellung einer injizierbaren Arzneimittelformulierung, umfassend die folgenden Schritte:
a) Bereitstellen eines Arzneimittelmisch- und -injektionssystems (200) nach einem der Ansprüche 10 bis 12;
b) Aktivieren des Arzneimittelmisch- und -injektionssystems (200) derart, dass der Adapter (100) aus der Ruheposition in die aktivierte Position übertragen wird;
c) Prüfen, ob das Ventil (121) des Adapters (100) des wie in Schritt a) bereitgestellten Arzneimittelmisch- und -injektionssystems (200) in seiner ersten Position ist, und wenn nicht, Bringen des Ventils (121) in die erste Position;
d) Übertragen der injizierbaren Flüssigkeit aus dem Behälter (115) in das Fläschchen (113), was zu einem Gemisch aus dem Arzneimittel und der injizierbaren Flüssigkeit führt;
e) Übertragen des in Schritt d) entstandenen Gemischs aus dem Fläschchen (113) zurück in den Behälter (115);
f) Prüfen des in Schritt e) entstandenen Gemischs, ob das Arzneimittel mit der injizierbaren Flüssigkeit bis zu einem gewünschten Maß an Homogenität gemischt ist, und wenn nicht, Wiederholen der Schritte d) und e) und/oder Schütteln des Arzneimittelmisch- und -injektionssystems (200);
g) sobald das Arzneimittel in dem entstandenen Gemisch in dem gewünschten Maß mit der injizierbaren Flüssigkeit gemischt und gemäß Schritt e) an den Behälter (115) übertragen ist, Bringen des Ventils (121) in die zweite Position, was die zweite Fluidströmung ermöglicht;
h) Übertragen des entstandenen Gemischs aus dem Behälter (115) an die Auslassöffnung (122).

14. Verfahren nach Anspruch 13, wobei Schritt a) die folgenden Schritte umfasst
- Bereitstellen eines Adapters (100) nach einem der Ansprüche 1 bis 9,
- Verbinden eines Fläschchens (113), das einen Deckel (114) umfasst und eine erste zentrale Längsachse aufweist, mit der ersten Öffnung (111) des Adapters (100),
- Verbinden eines Behälters (115), der eine injizierbare Flüssigkeit umfasst und eine zweite Längsachse aufweist, mit der zweiten Öffnung (112) des Adapters (100), und
- optionales Prüfen, ob der Adapter (100) in seiner Ruheposition ist, und wenn nicht, Bringen des Adapters (100) in die Ruheposition.

15. Verfahren nach Anspruch 13 oder 14, wobei das in Schritt a) bereitgestellte Arzneimittelmisch- und -injektionssystem (200) derart angepasst ist, dass es möglich ist, den Aktivierungsschritt b) mit einer Hand auszuführen.

## Revendications

1. Adaptateur (100) destiné à un système de mélange et d'injection de médicament (200), ledit adaptateur comprenant
- une première sous-unité (110) comprenant
- une première ouverture (111) adaptée pour contenir un flacon (113), ledit flacon (113) comprenant un couvercle perforable (114), et présentant un premier axe central longitudinal, et
- une deuxième ouverture (112) adaptée pour recevoir un réservoir (115) comprenant un liquide injectable, et présentant un deuxième axe central longitudinal,
les première et deuxième ouvertures (111, 112) étant agencées de manière à maintenir le flacon (113) et le réservoir (115) dans une position dans laquelle les premier et deuxième axes centraux longitudinaux sont positionnés suivant un angle inférieur à 75° ;
- une deuxième sous-unité (120) comprenant un orifice de sortie (122) ; et
- une première canule (134) adaptée pour perforer le couvercle (114) du flacon (113) ;
les première et deuxième sous-unités (110, 120) pouvant s'engager l'une avec l'autre, de telle sorte que l'adaptateur (100) ait
- une position de repos dans laquelle les première et deuxième sous-unités (110, 120) sont reliées de manière amovible ou non amovible et la première canule (134) ne perfore pas le couvercle (114) du flacon (113) ; et
- une position activée dans laquelle les première et deuxième sous-unités (110, 120) sont décalées l'une vers l'autre et se trouvent dans une position dans laquelle la première canule (134) perfore le couvercle (114) du flacon (113),
**caractérisé en ce que** :
- l'adaptateur (100) comprend au moins un conduit d'adaptateur interne (131) permettant une liaison fluidique entre la première ouverture (111), la deuxième ouverture (112) et l'orifice de sortie (122) ;
- la deuxième sous-unité (120) comprend en outre une valve (121) adaptée pour commuter au moins entre
- une première position permettant un premier écoulement fluidiue entre la première ouverture (111) et la deuxième ouverture (112), et bloquant la liaison fluidique à l'orifice de sortie (122), et
- une deuxième position permettant un deuxième écoulement fluidique entre la deuxième ouverture (112) et l'orifice de sortie (122), et bloquant la liaison fluidique à la première ouverture (111) ;
la première canule (134) est positionnée entre la valve (121) et la première ouverture (111) et est en outre adaptée pour permettre une liaison fluidique entre le flacon (113) et l'au moins un conduit d'adaptateur interne (131) lors de la perforation du couvercle (114) du flacon (113), et
**caractérisé en outre en ce que** :
- le réservoir (115) sst fournie sous la forme d'une seringue (115a) ; et
- l'orifice de sortie (122) est adapté de telle sorte qu'il puisse être utilisé pour effectuer une injection après avoir fixé une aiguille d'injection à l'orifice de sortie (122).

2. Adaptateur (100) selon la revendication 1, l'adaptateur (100) comprenant des moyens de guidage (132) destinés à guider la première sous-unité (110) et la deuxième sous-unité (120) lors de leur décalage l'une vers l'autre de la position de repos à la position activée et/ou la première ou la deuxième sous-unité (110, 120) comprenant au moins un moyen de verrouillage de sous-unité (133) destiné à l'engagement de la première sous-unité (110) avec la deuxième sous-unité (120).

3. Adaptateur (100) selon la revendication 1 ou 2, dans la position de repos les première et deuxième sous-unités (110, 120) étant reliées de manière non amovibles, ou dans la position de repos et dans la position activée les première et deuxième sous-unités (110, 120) étant reliées de manière non amovibles.

4. Adaptateur (100) selon l'une quelconque des revendications précédentes, les premier et deuxième axes centraux longitudinaux étant positionnés suivant un angle compris entre 50° et 0°, de préférence entre 20° et 0°, plus préférablement entre 10° et 0°.

5. Adaptateur (100) selon l'une quelconque des revendications précédentes, la première ouverture (111) étant équipée d'un moyen de verrouillage de flacon (116) destiné à relier de manière non amovible le flacon (113) à la première ouverture (111).

6. Adaptateur (100) selon l'une quelconque des revendications précédentes, l'adaptateur, de préférence la deuxième sous-unité, comprenant en outre au moins un site dédié destiné à l'appui d'un doigt lors du passage de l'adaptateur (100) de la position de repos à la position activée.

7. Adaptateur (100) selon l'une quelconque des revendications précédentes, l'adaptateur (100) pouvant être stérilisé.

8. Adaptateur (100) selon l'une quelconque des revendications précédentes, l'adaptateur (100) comprenant une deuxième canule (135) positionnée entre la valve (121) et la deuxième ouverture (112), ladite deuxième canule (135) étant adaptée pour perforer le couvercle du réservoir (115) et pour permettre une liaison fluidique entre le réservoir (115) et l'au moins un conduit d'adaptateur interne (131).

9. Adaptateur (100) selon l'une quelconque des revendications précédentes, la première sous-unité (110) comportant un filtre perméable aux gaz et imperméable aux liquides, ledit filtre étant conçu pour compenser et/ou équilibrer la pression.

10. Système de mélange et d'injection de médicament (200) comprenant
l'adaptateur (100) selon l'une quelconque des revendications 1 à 9,
un flacon (113) comprenant un couvercle perforable (114) et présentant un premier axe central longitudinal, et
un réservoir (115) comprenant un liquide injectable et présentant un deuxième axe central longitudinal.

11. Système de mélange et d'injection de médicament (200) selon la revendication 10, le système d'injection état pré-assemblé avant utilisation.

12. Système de mélange et d'injection de médicament (200) selon la revendication 11, le système d'injection pouvant être stérilisé à l'état pré-assemblé.

13. Procédé de préparation d'une formulation de médicament injectable, ledit procédé comprenant les étapes subséquentes suivantes :
a) fournir un système de mélange et d'injection de médicament (200) selon l'une quelconque des revendications 10 à 12 ;
b) activer le système de mélange et d'injection de médicament (200) de telle sorte que l'adaptateur (100) soit transféré de la position de repos à la position activée ;
c) vérifier si la valve (121) de l'adaptateur (100) du système de mélange et d'injection de médicament (200) tel que fourni à l'étape a) soit dans sa première position, sinon amener la valve (121) dans la première position ;
d) transférer le liquide injectable du réservoir (115) au flacon (113), ce qui a pour résultat de mélanger le médicament et le liquide injectable ;
e) transférer le mélange résultant de l'étape d) du flacon (113) dans le réservoir (115) ;
f) vérifier le mélange résultant de l'étape e) pour savoir si le médicament est mélangé au liquide injectable avec l'homogénéité souhaitée, sinon répéter les étapes d) et e), et/ou agiter le système de mélange et d'injection de médicament (200) ;
g) une fois que, dans le mélange résultant, le médicament est mélangé au liquide injectable dans la mesure souhaitée, et transféré vers le réservoir (115) selon l'étape e), amener la valve (121) dans la deuxième position, ce qui permet le deuxième écoulement fluidique ;
h) transférer le mélange résultant du réservoir (115) vers l'orifice de sortie (122).

14. Procédé selon la revendication 13, l'étape a) comprenant les étapes suivantes
- fournir un adaptateur (100) selon l'une quelconque des revendications 1 à 9,
- relier à la première ouverture (111) de l'adaptateur (100) un flacon (113) comprenant un couvercle (114) et présentant un premier axe central longitudinal,
- relier à la deuxième ouverture (112) de l'adaptateur (100) un réservoir (115) comprenant un liquide injectable et présentant un deuxième axe longitudinal, et
- éventuellement vérifier si l'adaptateur (100) est dans sa position de repos, sinon amener l'adaptateur (100) dans la position de repos.

15. Procédé selon les revendications 13 ou 14, le système de mélange et d'injection de médicament (200) fourni à l'étape a) étant adapté de telle sorte qu'il soit possible de réaliser l'étape d'activation b) d'une seule main.
